# EUROPEAN PATENT APPLICATION

(11) **EP 0 689 766 A1**
(43) Date of publication of application: **03.01.1996**
(21) Application number: 95109726.0
(22) Date of filing: 22.06.1995
(51) Int. Cl.: A01M 1/20, A61L 9/03

(54) **Apparatus for vaporizing insecticidal solutions and the like**

(30) Priority: 28.06.1994 IT BO940118 U
(71) Applicant: FALP S.r.l., I-40060 Passo Segni Baricella, Bologna (IT)
(72) Inventor: Falchieri, Roberto, I-40060 Passo Segni Baricella, Bologna (IT)
(74) Representative: Modiano, Guido, Dr.-Ing.

(57) **Abstract**

An apparatus for vaporizing insecticidal solutions and the like placed in a container from which a portion af an element made of absorbing material or wick protrudes, comprising a casing (1) that is adapted to be associated with said container and forms the seat for a printed circuit (15) provided with heating means (17) that are adapted to heat said wick.

## Description

The present invention relates to an apparatus for vaporizing insecticidal solutions and the like.

Heating devices which cause the evaporation of vaporizable solutions containing active insecticidal or deodorant substances are currently known. These vaporizable solutions are placed in a container in which an element made of absorbing material, or wick, is introduced; said wick dips in the solution. The absorbing element is commonly made of highly porous materials, such as paper, felt and the like, or of low-porosity materials, such as ceramic and the like.

In order to achieve evaporation of the solution, a portion of the absorbing element protruding from the container of the vaporizable solution is moved close to the heating means of the apparatus, which is generally powered electrically.

For this purpose, the apparatus has an appropriate electric circuit that is generally manufactured separately by specialized personnel and is subsequently assembled together with the other parts of the apparatus. This solution obviously entails low productivity and high labor costs.

The fact is furthermore observed that said power supply circuit usually includes various electric components that are connected by wires, which are cumbersome during assembly besides being a source of possible hazard.

The aim of the present invention is to solve the above mentioned problem by providing an apparatus for vaporizing insecticidal solutions and the like allowing to rapidly and easily assemble the various parts, particularly the components for supplying electric power to the heating means.

Within the scope of this aim, an object of the present invention is to provide a device that is simple in concept, safely reliable in operation, and versatile in use.

This aim, this object and other, are achieved, according to the invention, by an apparatus for vaporizing insecticidal solutions and the like placed in a container from which a portion of an element made of absorbing material or wick protrudes, characterized in that it comprises a casing that is adapted to be associated with said container and forms the seat for a printed circuit provided with heating means that are adapted to heat said wick.

The features of the invention will become apparent from the detailed description of a preferred embodiment of the apparatus for vaporizing insecticidal solutions and the like, illustrated only by way of non-limitative example in the accompanying drawings, wherein:

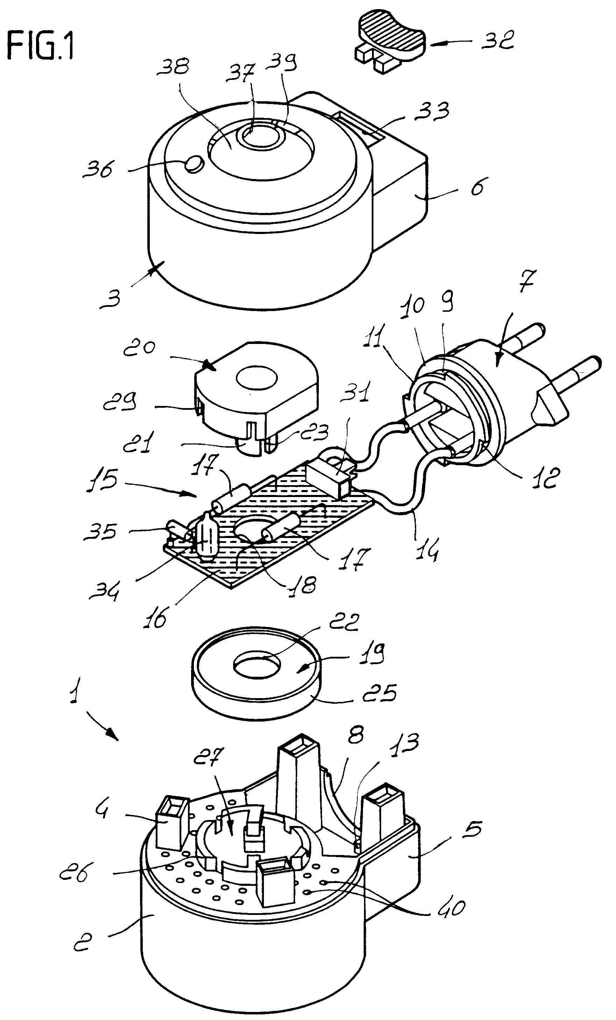

figure 1 is an exploded perspective view of the apparatus according to the invention;
figure 2 is a partial sectional view of the apparatus, taken along a vertical plane;
figure 3 is a corresponding bottom plan view, showing in detail the area for accommodating said heating means;
figure 4 is a partial exploded perspective view of a further embodiment of the apparatus according to the invention;
figure 5 is a sectional view of a detail of the apparatus shown in figure 4.

Referring to the above figures, the reference numeral 1 generally designates the casing of the apparatus according to the invention, which is preferably made of molded plastics. The casing 1 includes a cylindrical body 2, which is internally hollow and open at the bottom for coupling to a container for the vaporizable solution, which is constituted in practice by a small glass bottle and is not shown in the drawing; a portion of an element made of absorbing material, or wick, that dips in the solution protrudes from said container.

A lid 3 is adapted to be associated with the body 2 and has a correspondingly cylindrical shape. The lid 3 has a plurality of pins, not shown in the drawings, that protrude internally and are adapted for snap-together coupling to a corresponding plurality of tubular couplings 4 that protrude upward from the body 2.

The body 2 and the lid 3 of the casing 1 laterally respectively form the complementary lower and upper portions 5 and 6 of a box-like bracket that is meant to provide rotatable coupling to an electric plug 7. The portions 5 and 6 of said bracket are in fact frontally provided with respective halves of a circular opening 8 that is meant to engage an annular groove 9 formed between two circular shoulders 10 and 11 provided to the rear of the plug 7.

The internal shoulder 11 is provided with two oppositely arranged recesses 12 that are shaped so as to form an arc that covers substantially 90^{o}. Respective teeth 13 formed inside the portions 5 and 6 are adapted to cooperate with the recesses 12 and to act as stroke limiters for the angular rotation of the electric plug 7. In practice, the plug 7 can rotate between two positions at right angles with respect to the casing 1, so as to allow to arrange the apparatus vertically regardless of the orientation of the electric wall outlet.

The electric plug 7 is connected by means of two short wires 14 to a printed circuit which is generally designated by the reference numeral 15 and is meant to supply the heating means of the apparatus. The printed circuit 15 is constituted by a board 16 on which the electric components of said circuit are mounted; in particular, said electric components include two electric resistors 17 that are adapted to provide the above mentioned heating means.

The electric resistors 17 are arranged along opposite sides of the board 16, in diametrically opposite positions with respect to a hole 18 formed centrally on said board. The wick protruding from the container of the vaporizable solution is meant to enter through said hole 18, as specified hereinafter.

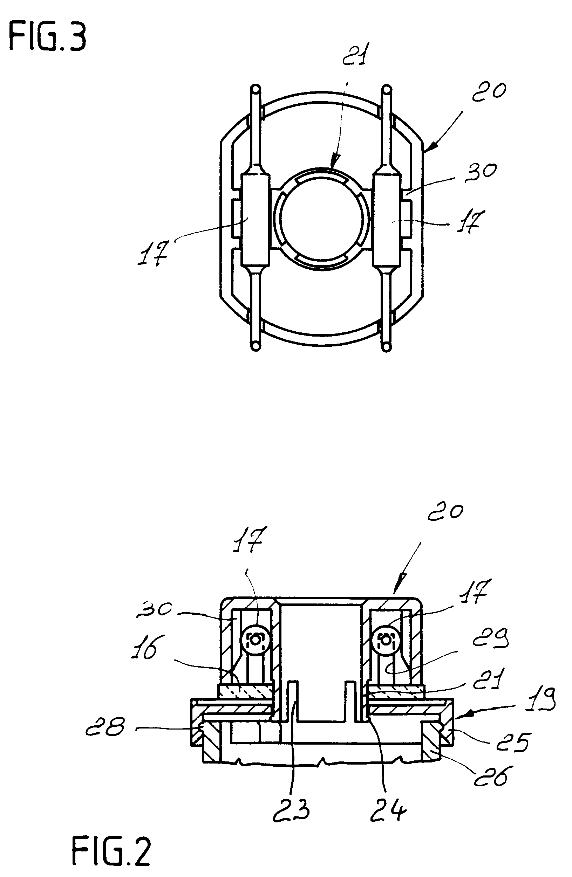

The board 16 of the printed circuit 15 is adapted to be secured between a disk 19 and a cap 20, by means of which it is meant to be mounted inside the casing 1 of the apparatus. The cap 20 forms, on the side provided with the concavity, a tubular stem 21 which, by passing through the hole 18 of the board 16, is adapted to fit in a corresponding hole 22 that is formed centrally with respect to the disk 19. A portion of the tubular stem 21 is provided with a plurality of longitudinal slots 23 that are adapted to facilitate its elastic insertion through said holes 18 and 22, whose diameter substantially matches that of said stem 21; the engagement of the stem 21 with the disk 19 is furthermore ensured by an annular tooth 24 formed at the inlet of the stem 21 (see figure 2).

The disk 19 is circular and is peripherally provided with a rim 25, by means of which it is adapted to engage in turn on a plurality of raised portions 26 that protrude from the upper surface of the body 2, at the edge of a central opening 27 of said body 2. The engagement of the disk 19 to the body 2 is ensured by a tooth 28 that is formed externally by the raised portions 26 and is adapted to engage, in a snap-together manner, a corresponding annular groove formed on the inner surface of the rim 25.

The cap 20 is shaped so as to cover the electric resistors 17 when assembled. For this purpose, the cap 20 is provided with oppositely arranged slits 29 for the passage of the leads of the resistors 17.

It should be noted that the electric resistors 17 are retained in assembled configuration between the tubular stem 21 and pairs of ridges 30 formed inside the cap 20. The resistors 17 accordingly transmit heat to the cap 20, which is made of heat-resistant plastics like the disk 19, directly by contact. The tubular stem 21 thus forms a chamber for heating the wick protruding from the container of the vaporizable solution.

The apparatus can be conveniently provided with a switch 31 mounted on the board 16 outside the cap 20; the switch 31 can be actuated by means of a button 32 that is mounted at an upper opening 33 of the portion 6 of the lid 3. Of course, if this switch is omitted, the apparatus switches on automatically by inserting the plug 7 in the electric outlet.

It is also possible to provide, on the board 16 of the printed circuit, a lamp 34 provided with a corresponding resistor 35 that is adapted to act as power-on indicator. The indicator is visible through a hole 36 that is appropriately closed by an element made of transparent material and is formed on the upper surface of the lid 3.

The upper surface of the lid 3 furthermore has a hole 37, which is arranged at the center of a dome 38 and is aligned with the tubular stem 21, in assembled configuration, to allow the discharge of the vaporized solution. The dome 38 is surrounded by vapor venting slits 39; for the same purpose, the upper surface of the body 2 is provided with a plurality of appropriately distributed small holes 40.

In a more advanced version, the apparatus can furthermore be provided with a fuse element, which is mounted on the board 16 inside or outside the cap 20 and is not shown in the drawings. Said fuse is meant to interrupt the supply of electric power to the circuit when a safety temperature is exceeded.

To conclude, the described apparatus allows to assemble the electric components very easily and quickly, since said components are collected on the board 16 of the printed circuit 15. Said printed circuit is secured between the disk 19 and the cap 20, and is then associated with the body 2 of the casing by engaging said disk 19 on the raised portions 26 of the body 2.

It should be noted that the operations for assembling the printed circuit 15 are performed simply by means of elements that engage in a snap-together manner and therefore do not require screw coupling means and the like.

Moreover, said operations for assembling the printed circuit 15 can be easily performed by means of appropriate automatic devices. This of course allows to achieve high productivity at proportionally reduced costs.

The apparatus furthermore provides optimum heating of the wick, for vaporizing the solution, by virtue of the two electric resistors 17 that are arranged symmetrically with respect to the seat of said wick, formed by the tubular stem 21, and are in direct contact with said stem for transmitting heat.

The electric resistors 17 have a precisely defined position by virtue of their mounting on the board 16 and of their engagement inside the cap 20, so as to ensure the desired heating conditions.

An additional advantage offered by the apparatus according to the invention is constituted by the possibility to equip the products put on sale in different manners, by adding the switch 31, the indicator lamp 34, and a fuse to the basic model. Said components are all pre-mounted on the board 16 of the printed circuit, and therefore the previously described assembly operations are not modified.

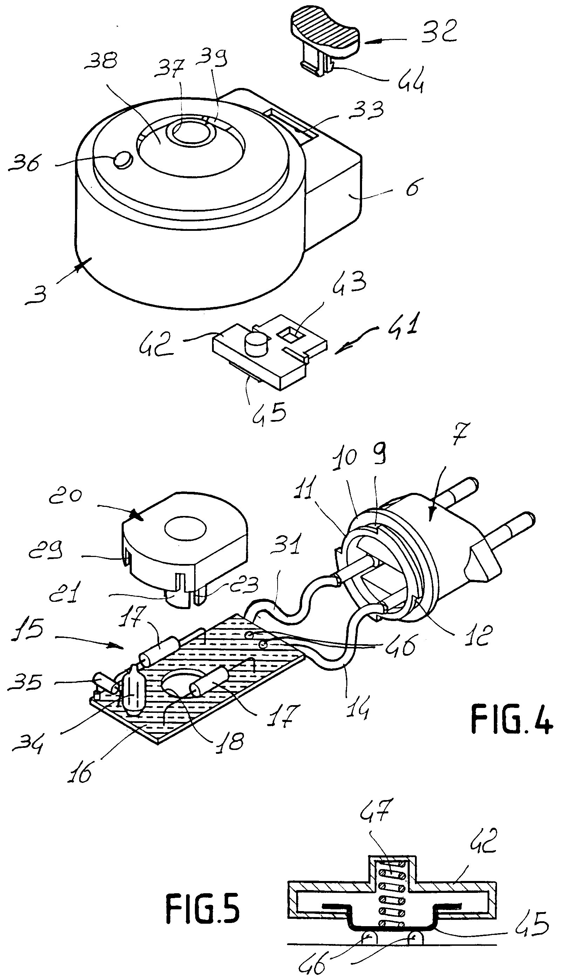

Figures 4 and 5 illustrate a further embodiment of the apparatus, in which a switch 41 is provided to switch on the apparatus, said switch being operatable by means of said button 32. Said switch 41 has a slider 42 made of insulating material that is appropriately guided, inside the portion 6 of the lid 3, longitudinally with respect to the opening 33; the slider 42 has a hole 43 in which a fork-like coupling element 44, which protrudes from the button 32, is inserted in a snap-together manner.

An electric terminal 45 is coupled to the slider 42 and is adapted to provide, in an operating position, the connection between two contacts 46 of the electric power supply circuit that are arranged on the board 16. The terminal 45 is actuated elastically, at right angles to the board 16, by a helical spring 47 accommodated inside said slider 42.

Accordingly, by acting on the button 32, it is possible to alternately move the slider 42 between a position for switching on the apparatus, in which the terminal 45 closes the electric power supply circuit by connecting the contacts 46 (see figure 5), and a power-off position, in which said power supply is interrupted.

In the practical embodiment of the invention, the materials employed, as well as the shapes and the dimensions, may be any according to the requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Apparatus for vaporizing insecticidal solutions and the like placed in a container from which a portion of an element made of absorbing material or wick protrudes, characterized in that it comprises a casing (1) that is adapted to be associated with said container and forms the seat for a printed circuit (15) provided with heating means (17) that are adapted to heat said wick.

2. Apparatus according to claim 1, characterized in that said heating means (17) comprise two electric resistors mounted symmetrically along the sides of a board (16) of said printed circuit (15), on opposite sides with respect to a hole (18) which is formed in said board (16) and through which said wick passes in operating conditions.

3. Apparatus according to claim 1, characterized in that said printed circuit (15) is adapted to be secured between a disk (19), meant to be associated with said casing (1), and a cap (20) for covering said heating means (17).

4. Apparatus according to claim 3, characterized in that said cap (20) forms, on the side provided with the concavity, a tubular stem (21) which, by passing through said hole (18) of the board (16) of said printed circuit (15), is adapted to enter and engage elastically in a corresponding hole (22) formed centrally with respect to said disk (19).

5. Apparatus according to claim 3, characterized in that said disk (19) is peripherally provided with a rim (25) by means of which it is adapted to engage, in a snap-together manner, on a plurality of raised portions (26) protruding from the upper surface of a lower body (2) of said casing (1), at the edge of a central opening (27) of said body (2).

6. Apparatus according to claim 3, characterized in that said cap (20) forms, on opposite sides with respect to a tubular stem (21) for coupling to said disk (19), seats (29,30) for accommodating two electric resistors (17) that are mounted on said printed circuit (15) and constitute said heating means, said electric resistors (17) being placed, in operating conditions, in contact with said tubular stem (21) to transmit heat.

7. Apparatus according to claim 1, characterized in that said printed circuit (15) is provided with a fuse element that is meant to interrupt the supply of electric power to said printed circuit (15) when a safety temperature is exceeded.

8. Apparatus according to claim 1, characterized in that said casing (1) comprises a cylindrical body (2), which is internally hollow and open at the bottom for coupling to said container, said printed circuit (15) being adapted to be coupled to the upper surface of said cylindrical body (2), and a lid (3), which has a correspondingly cylindrical shape and is adapted to be associated above said cylindrical body (2), and in that said cylindrical body (2) and said lid (3) form, in a lateral area, respective complementary portions (5,6) that are adapted to form a rotatable coupling seat for an electric plug (7) for supplying power to said printed circuit (15).

9. Apparatus according to claim 1, characterized in that it comprises a power-on switch (41) provided with a slider (42) that is slidingly guided inside said casing (1) and is provided with an elastically actuated electric terminal (45) adapted to connect, in a power-on operating position, two contacts (46) of a circuit for supplying electric power to said heating means (17).
